# EUROPÄISCHE PATENTANMELDUNG

(11) **EP 1 609 420 A1**
(43) Veröffentlichungstag der Anmeldung: **28.12.2005**
(21) Anmeldenummer: 05010002.3
(22) Anmeldetag: 20.07.1998
(51) Int. Cl.: A61B 6/04

(54) **Vorrichtung und Verfahren zur Fixierung und/oder Komprimierung und/oder Abformung vo Körper(-teilen)**

(30) Priorität: 19.07.1997 DE 19731040
(62) Teilanmeldung aus: 98943744.7
(71) Anmelder: Müller, Christian, 86863 Langenneufnach (DE); Vogele, Michael, 86830 Schwabmünchen (DE)
(72) Erfinder: Müller, Christian, 86863 Langenneufnach (DE); Vogele, Michael, 86830 Schwabmünchen (DE)
(74) Vertreter: Fiener, Josef

(57) **Zusammenfassung**

Zur Fixierung und/oder Komprimierung und/oder Abformung von Körper-(teilen), insbesondere des menschlichen Körpers, wird eine Vorrichtung vorgeschlagen, die eine Körperauflagefläche (1), mindestens ein Außenelement (2), welches frei auf der Körperauflagefläche (1) positionierbar ist und mit der Körperauflagefläche (1) eine den Körper umschließende Unterdruckkammer (6) bildet, und mindestens eine Absaugöffnung (3), welche mit der Unterdruckkammer (6) in Verbindung steht, aufweist.

## Beschreibung

Die Erfindung betrifft eine Vorrichtung und ein Verfahren zur Fixierung von Körper/Körperteilen, insbesondere des menschlichen Körpers bzw. von menschlichen Körperteilen.

In vielen Bereichen der Humanmedizin oder der medizinischen Forschung und Technik ist eine sichere Fixierung des Patienten oder einer Untersuchungsperson erforderlich. Von größter Bedeutung ist dies insbesondere auf dem Gebiet der diagnostischen Anwendung (Radiologie), der Strahlentherapie oder bei operativen/chirurgischen Eingriffen (Neurochirurgie, HNO, usw.) aber auch bei (akuten oder permanenten) prä- oder postoperativen Versorgungen von Wunden/Verletzungen. Durch die Einbeziehung der Computertechnologie in Diagnose und Therapie sind die Anforderungen an Genauigkeit und Reproduzierbarkeit sowohl bei der Festlegung eines stereotaktischen Rahmensystems im oder am menschlichen Körper, als auch bei der Fixierung des Körpers selbst, gestiegen.

Als Stand der Technik sind folgende Fixationsmethoden bekannt:

### a) Fixation des Körpers mit Klebebändern oder Manschetten:

Der Körper des Patienten liegt auf einer Schaumstoffunterlage. Quer über den Körper gespannte Bänder fixieren den Patienten auf diese Unterlage. Nachteilig sind dabei folgende Punkte:
- Durch starken Zug der Klebebänder kann es zu Eindrücken, Druckstellen, Verschiebungen und/oder Hautschwellungen kommen;
- nach Abnahme der Halteelemente (Klebebänder) ist eine erneute Repositionierung in genau gleicher Lage kaum mehr möglich, was besonders bei stereotaktischen Operationen und in der Strahlentherapie nachteilig ist;
- der Körper ist nicht ausreichend fixierbar; besonders in seitlicher Richtung (nach lateral) ist die Beweglichkeit zu wenig einschränkbar oder definierbar.

### b) Fixation des Körpers durch Verschraubung im Knochen:

Der Körper des Patienten wird an mehreren Stellen über einen Metallrahmen verschraubt. Nachteilig ist dabei folgendes:
- Die Verschraubung am Knochen stellt eine invasive Methode dar und ist somit nur bei bestimmten Indikationen möglich und gerechtfertigt;
- die psychische Belastung des Patienten ist erheblich;
- die Methode ist nur auf bestimmte Lagerungen des Patienten anwendbar und behindert den Operateur;
- eine Fixation der Weichteile (Muskel, Bänder, Bindegewebe) ist praktisch nicht möglich.

### c) Fixation durch Schalungen:

Hierbei wird der Patient auf einer Art "Luftmatratze" gelegt, welche mit Schaumstoffkügelchen gefüllt ist. Durch Absaugen der Luft in dieser Matratze verfestigt sich diese durch Aneinanderlegen der Schaumstoffkügelchen. Dabei wird die Vakuummatratze dem Körper im ersten schritt angepaßt und im zweiten Schritt abgesaugt. Durch diese Methode erhält man einen Abdruck des Körpers. Nachteilig ist hierbei:
- die üblicherweise verwendeten "Matratzen" garantieren zwar eine Ruhigstellung, aber keine Fixation. Bei unkooperativen oder narkotisierten Patienten ist eine ausreichende Ruhigstellung praktisch nicht möglich;
- oftmals entsteht ein ungenügender Abdruck des Patienten, da sich die Matratze praktisch nicht an allen Körperpartien exakt anlegen läßt;
- durch Faltenbildung oder zu starkes Andrücken entstehen oftmals Druckstellen, welche vor allem bei narkotisierten Patienten zu Druckstellen und Gewebeverletzungen führen können.

Andere Abdruckmaterialien wie Schienen, thermoplastisches Material, oder Kunstoffabdrucke, Gipse, etc. weisen ähnliche Nachteile auf. Zusätzlich sind diese Methoden noch mit erheblichem finanziellen oder zeitlichen Aufwand verbunden und werden deshalb nur für Langzeitanwendungen verwendet.

Der Erfindung liegt deshalb die Aufgabe zugrunde, eine Vorrichtung und ein Verfahren zur Fixierung/Abformung/Kompression des menschlichen Körpers bzw. von Körperteilen zu schaffen, welche die genannten Nachteile vermeidet, einfach in Aufbau und Anwendung und dabei in hohem Maße patientenschonend ist; die Vorrichtung soll darüber hinaus die exakte Anbringung von Eichpunkten (sog. Markern) und eine optimale Zugänglichkeit zu Operationsgebieten ermöglichen.

Die Aufgabe wird gelöst mit einer Vorrichtung gemäß den Merkmalen des Anspruches 1 bzw. den zugeordneten Verfahren. Bevorzugte Weiterbildungen sind Gegenstand der Unteransprüche.

Im Gegensatz zu den eingangs beschriebenen Vorrichtungen bzw. Verfahrensweisen erfolgt bei der vorgeschlagenen Vorrichtung bzw. den Verfahren die Fixierung des menschlichen Körpers im wesentlichen durch Unterdruckkräfte. Über einer (flexiblen/starren) Körperauflagefläche mit Absaugöffnungen wird ein deckenartiges Außenelement gelegt, welches in seinem über den Körper (des Patienten) hinausragenden Randbereich zumindest weitgehend luftdicht an der Körperauflagefläche angesaugt ist. Damit entsteht eine geschlossene Unterdruckkammer, so daß der Körper relativ zu der Auflagefläche exakt und unverschiebbar in allen Richtungen fixiert ist.

Der Patient legt sich hierzu in bevorzugter Ausführung einfach auf die Körperauflagefläche und wird mit dem folienartigen Außenelement "zugedeckt". Der Patient oder sein(e) Körperteil(e) sind nun von dieser beutelartigen Struktur umgeben. Durch "Schlupflöcher", können Teile des Körpers (Arme, Beine, Kopf, Rumpf) die Luft- oder Vakuumkammer verlassen und damit unfixiert bleiben, wobei diese Öffnungen mittels luftdichter Ausführung z.B. Gummibündchen oder luftdichten Manschetten abgegrenzt sind. Nun wird die Luft aus dem Raum zwischen Körperauflagefläche und Außenelement abgesaugt und der Patient in der so entstandenen Unterdruckkammer fixiert.

Das beschriebene Außenelement bzw. die Körperauflagefläche ist in bevorzugter Ausführung aus strukturverstärktem, leicht dehnbarem Kunststoffmaterial oder eine Metallfolie gebildet und kann sich dadurch den anatomischen Strukturen des Körpers genau anpassen. Durch die entsprechende Wahl der Materialstärke und/oder Erhöhung des Unterdrucks im Vakuumraum kann eine gesteigerte Fixation bzw. sogar Kompression des Körpers (des Patienten) realisiert werden, so daß das folienartige Außenelement wie eine zweite Haut straff über dem Körper(-teil) anliegt.

Durch diese Fixierung mittels Unterdruck kann dabei sichergestellt werden, daß insbesondere nach mehrfacher Benutzung der Vorrichtung eine immer feste, exakt gleichbleibende Fixation ermöglicht wird.

Als Mittel zur Erzeugung von Unterdruck sind Vakuumpumpen mit einstellbarer Saugdruckhöhe, Schlauchverbindungen und Absperrventile anwendbar. Diese Einrichtungen können in ihrer Vakuum-Wirkung auch gesteuert/geregelt werden. Zur Unterstützung der Vakuum-Wirkung können durch Einbringung von Poren/Kanälen an dem Außenelement bzw. der Körperauflagefläche flache Hohlräume oder Unterdruckkanäle erzeugt werden, welche eine homogene Absaugung garantieren, beispielsweise mittels einer aus Stoßschutzverpackungen bekannten Luftpolsterfolie mit Noppen. Damit kann das vorstehend beschriebene geschlossene System auch als halboffenes System mit dauernder Luftabsaugung, insbes. zur Ermöglichung der Hautatmung bei längeren Operationen betrieben werden. Eine weitere Methode, die beschriebene Wirkung zu erreichen, ist, zwischen dem Außenelement und dem Patienten ein Netz/Fell/Schaumstoff oder ähnlich strukturiertes Material über- und/oder unterzulegen. Derartige Materialien gewähren durch die Vielzahl ihrer Luftkanäle/Poren in dieser Anwendung eine optimale Strömung der Luft in allen Körperregionen. Neben Luft können auch andere Medien zur Erzeugung des Unterdruckes verwendet werden. Eventuelle "Luftflußbarrieren", die durch die Absaugung erzeugt werden oder diese beeinträchtigen, werden somit verhindert. Druckmeßeinrichtungen dienen zudem der Kontrolle eines etwa gleichbleibenden Druck auf die Körperregionen.

Durch einen Sicherheitsknopf kann bei Bedarf der Patient selbst (z. B. bei Erbrechen, Panik) oder der Operateur (z. B. bei Komplikation während des Eingriffes) den Unterdruck unterbrechen (oder sogar den Unterdruck kurzzeitig in einen Überdruck umwandeln) und damit den Körper des Patienten sehr rasch und unkompliziert aus der Fixation lösen, da sich dann das Außenelement "schlagartig" von der Auflagefläche abhebt.

Das deckenartige Außenelement, bevorzugt in Art einer an sich bekannten Wärmeschutz-Alufolie, ist als Einwegartikel steril abpackbar, aber auch einfach zu reinigen und sterilisierbar, so daß die Betriebskosten gering bleiben, da alle wesentlichen Teile wiederverwendet werden können.

Neben der reinen Fixation/Komprimierung/Abformung können auch Zusatzgeräte (wie Führungseinrichtungen, optische Geräte, Zielvorriohtungen, etc.) durch mechanische/pneumatische oder magnetische Verankerung eingesetzt werden, da das deckenartige Außenelement praktisch keinen zusätzlichen Platz beansprucht. Durch Skalierungen oder ähnliche Einrichtungen ist daher eine reproduzierbare Anbringung mit hoher Genauigkeit möglich.

Der Körper(-teil) kann dabei so gelagert werden, daß für den behandelnden Arzt oder sonstige Behandlungsperson praktisch jede Körperregion vollständig zugänglich ist, insbesondere für chirurgische Eingriffe, wobei eine solche Alu- oder Kunststoff-Folie auch noch nach Anlegen des Unterdrucks mit einem Skalpell ohne Druckabfall geöffnet werden kann. Von besonderem Vorteil ist dabei eine ähnlich wie das deckenartige Außenelement aufgebaute und damit variabel positionierbare Zusatzfixierung zur zusätzlichen Sicherung des Patienten. Vor allem in weichen Regionen des Ober- und Unterbauches oder im Schulterbereich kann durch einfaches Einbringen von schienen- und stützelementen die Fixationswirkung erhöht werden. Die Anbringung dieser Schienen steigert die Repositioniergenauigkeit zusätzlich. Ebenso ist hierdurch eine Fixierung des Patienten, insbesondere bei unruhigen Patienten oder bei größeren Krafteinwirkungen von außen, wie bei Knochen- oder Knorpelbearbeitungen, gesichert.

Zudem ermöglicht diese Vakuumtechnik mit mindestens einem sich auf den Körper bzw. die Haut legenden Außenelement eine besonders gleichmäßige Druckverteilung und damit eine Entlastung des Patienten. Verletzungen sind dabei praktisch ausgeschlossen, auch bei narkotisierten Patienten, welche keine Schmerzangaben bei Druckschmerz machen können.

Besonders vorteilhaft ist auch die Anbringung von passiven oder aktiven Eichpunkten an dem Außenelement oder der Auflagefläche, die in der modernen Medizin Referenzpunkte und Bezugsebenen bei der praktischen Anwendung von bildgebenden Verfahren wie CT (Computertomographie) oder MRI (Kernspintomographie) festlegen. Die Fixiervorrichtung erlaubt es somit, Eichpunkte auf einem Teil der Vorrichtung (primär auf der Körperauflagefläche) anzubringen, die mit oder ohne Fixierung des Patienten in einem bildgebenden Verfahren als Bezugspunkte für weitere Maßnahmen, wie z.B. die Eichung zur Operationsvorbereitung, verwendet werden können.

Besteht das Ziel nicht nur in einer Fixation, sondern in einer reproduzierbaren Lagerung, so können zusätzlich vorgefertigte Schalungen, Bänder, Vakuummatratzen verwendet werden, welche die Reproduzierbarkeit erhöhen. Da durch das Absaugen der Körper (des Patienten), insbesondere bei konkaven Auflageflächen in die jeweils gleiche Position "wandert", kann eine hohe Repositioniergenauigkeit geschaffen werden. Dabei kann durch Gleitfolien der Widerstand zwischen Patient und Schalung bzw. zwischen Patient und Außenelement oder Körperauflagefläche besonders gering gehalten werden, so daß das Einnehmen der richtigen Position ohne hohen Kraftaufwand durch Scherkräfte erfolgt.

Eine Unterpolsterung erhöht zusätzlich den Komfort. Dabei kann durch variable Unterdruckregelung die Polsterwirkung bei Erhöhung des Unterdruckes partiell aufgehoben werden und die Fixation verstärkt werden und umgekehrt. Auch kann für unterschiedliche Körperregionen ein unterschiedlicher Unterdruck appliziert werden oder im Laufe einer Fixation variiert werden, beispielsweise um eine Massagewirkung zur Verstärkung der Durchblutung auszuüben.

Das Außenelement kann entweder druckdicht geschlossen sein, wie dies bei einer Aluminiumfolie der Fall ist, oder feine Poren/Kanäle enthalten, wodurch dann mittels eines permanenten "Flows" der erwünschte Unterdruck aufrecht erhalten wird. Die einströmende Luft kann in Wärme und Feuchtigkeitsgehalt reguliert werden, um damit eine gewünschte Abkühlung/Erwärmung oder Austrocknung/Anfeuchtung des fixierten Körpers zu erzielen. Ebenso ist es möglich, ergänzend oder statt der einströmenden Luft andere Gase oder auch Flüssigkeiten zu verwenden, die z.B. den Heilungsprozeß von Wunden beschleunigen. Es ist somit auch möglich, ein künstliches Klima unter dem angesaugten Außenelement und der so gebildeten Vakuumkammer zu erzeugen.

Die beschriebene Fixation kann entweder für den ganzen Körper angewendet werden oder auch nur für einzelne Körperteile. So können z.B. Knochenbrüche und komplizierte Frakturen anstatt mit Gipsverbänden mittels beschriebener Vakuumfixation ruhiggestellt werden. Bei einer bevorzugten Methode werden Schalungen oder Schienungen mit der gewünschten, zu erzielenden Anatomie mit in den Vakuumverband eingearbeitet. Das angelegte Vakuum bewirkt, daß eine homogene Anlage der Schalungen an den Körperteilen erzielt wird bzw. sich die Körperteile diesen Strukturen anlegen. Bei offenen Wunden oder großen Schnittverletzungen, die starke Narbenbildung zur Folge haben, bewirkt der Vakuumverband, daß sich die Wundränder aneinanderlegen und somit glattflächig schließen lassen. So kann in manchen Fällen auf eine Hautnaht an der Hautoberfläche verzichtet werden.

Ein weiterer Anwendungsfall ist das homogene Anlegen und Andrücken von Materialien und Geweben an die Hautoberfläche des Patienten. Hierbei wird das jeweilige Material oder Gewebe an die gewünschte stelle angebracht. Darüber wird dann das Außenelement als Vakuumverband gelegt. Wird nun die Luft abgesaugt, legt sich zwangsläufig das angebrachte Material an die Hautoberfläche. Anstatt der direkten Anbringung an der Hautoberfläche kann das Außenelement auch an andere Gewebearten und Strukturen aufgelegt werden.

Aussparungen im Außenelement oder in der Körperauflagefläche, welche mittels Randdichtungen luftdicht am Patienten anschließen, erlauben den direkten, optimalen Zugang zum Patienten für den Operateur. Dieses "Operationsfenster", insbesondere im Außenelement kann je nach Körperregion mit einem anatomisch geformten, strukturgebenden Element versehen sein, beispielsweise einem randverstärkten Gummiband. Der luftdichte Abschluß wird entweder durch die Eigenschaft der Außenhaut selbst, beispielsweise einer Innengummierung oder durch abdichtende Materialien (Klebstoff, Gummibänder, etc.) erzielt.

Im folgenden werden bevorzugte Ausführungsbeispiele anhand der Zeichnungen näher beschrieben und erläutert. Hierbei zeigen:

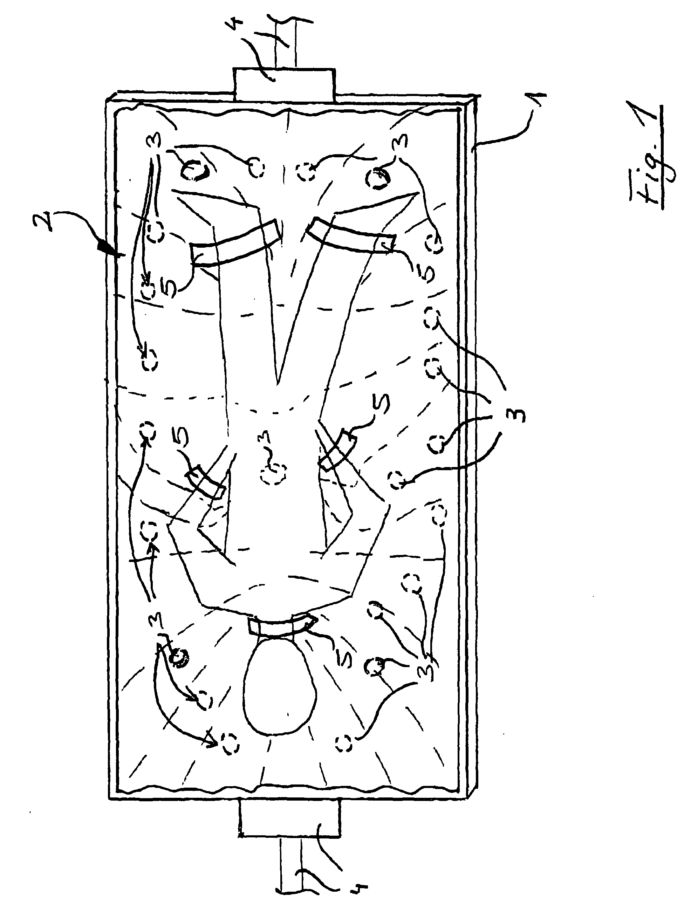

- Fig. 1: eine Vorrichtung zur Fixierung des Körpers eines Patienten in schematischer Draufsicht;

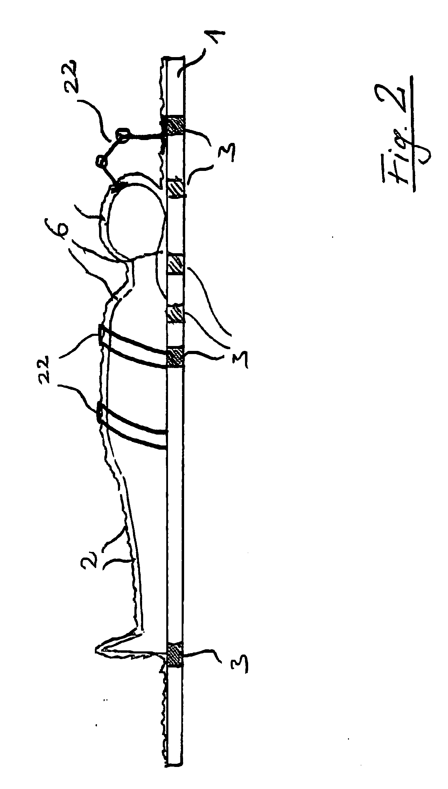

- Fig. 2: die Vorrichtung gemäß Fig. 1 in Seitenansicht;
- Fig. 3: die Vorrichtung gemäß Fig. 1 und 2 im Querschnitt;
- Fig. 4: eine bevorzugte Anbringung von Eichsystemen auf der Auflagefläche in Draufsicht;

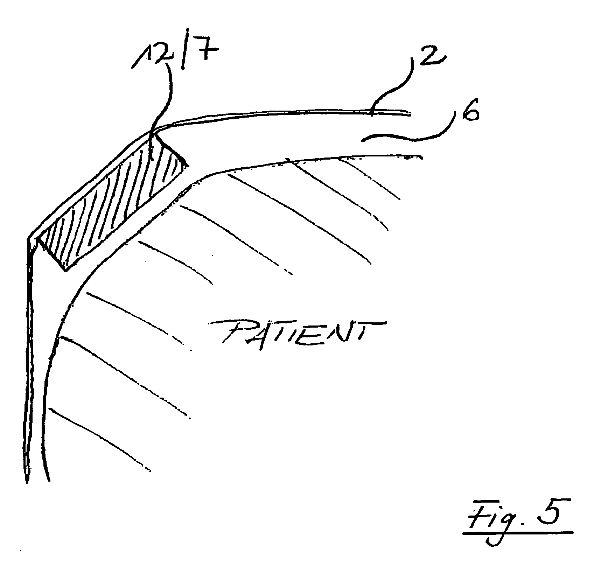

- Fig. 5: ein Patient mit einer Schienung im Querschnitt;
- Fig. 6: eine Schnittdarstellung einer Abformung im Vakuumverfahren;
- Fig. 7: eine Schnittdarstellung einer beidseitigen, flexiblen Patientenauflage bzw. Abdeckung mit einer Rahmenaufhängung;

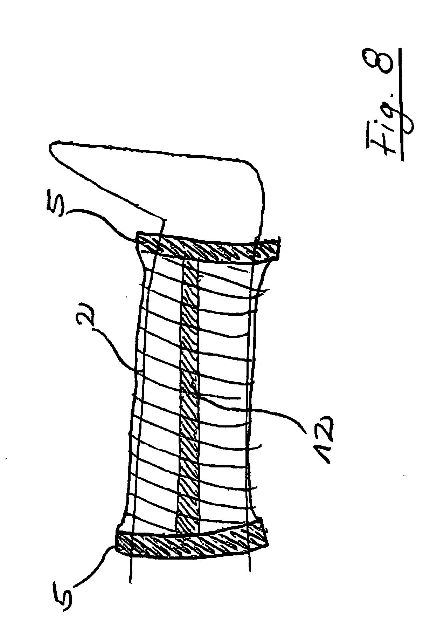

- Fig. 8: ein Vakuumverband mit einer Schienung in Seitenansicht;

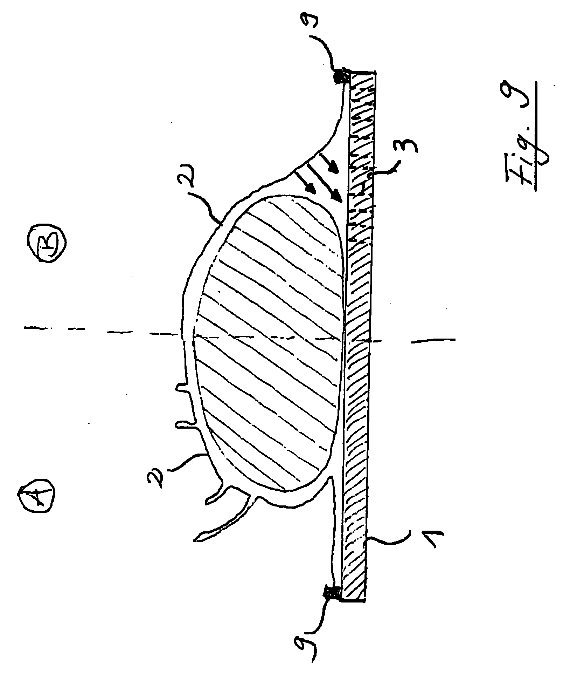

- Fig. 9: eine vergleichende Schemazeichnung zur Darstellung einer Fixation/ Abformung (A) und einer Kompression (B) eines Körpers im Querschnitt;
- Fig. 10: eine Schemazeichnung zur Darstellung der Absaugung mit einzelnen Ansaugkanälen (A) und Absaugung mit einer Absaugkammer (B) im Querschnitt;

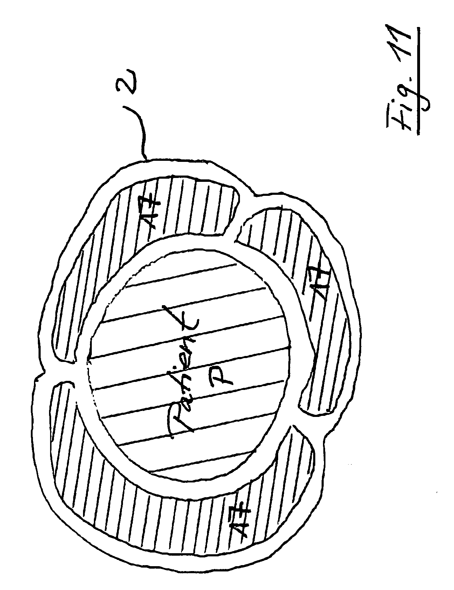

- Fig. 11: ein Vakuumverband mit Schalungen im Querschnitt;
- Fig. 12: eine schematische Darstellung einer Führungseinrichtung für Schalungs- und Schienungsalemente; und

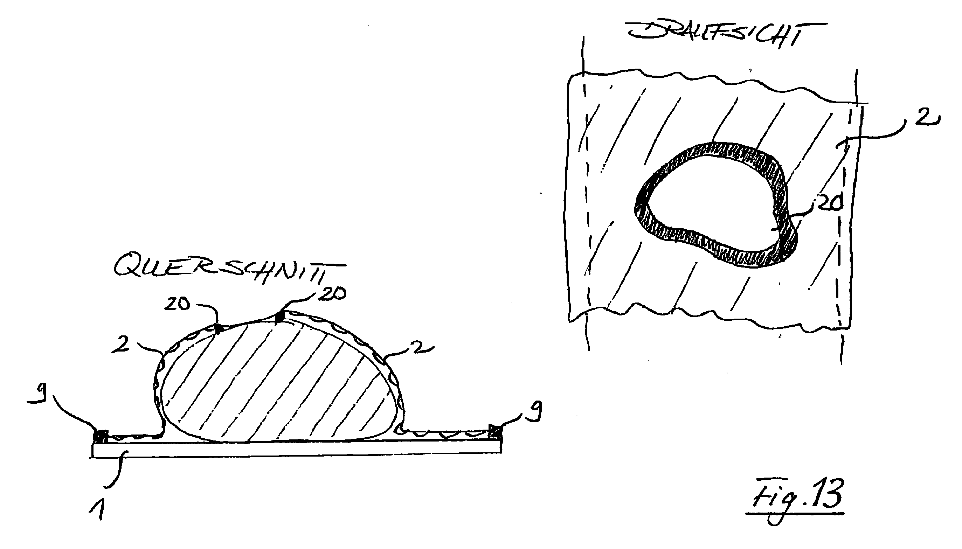

- Fig. 13: ein Operationsfenster in einem Außenelement im Querschnitt und in Draufsicht.

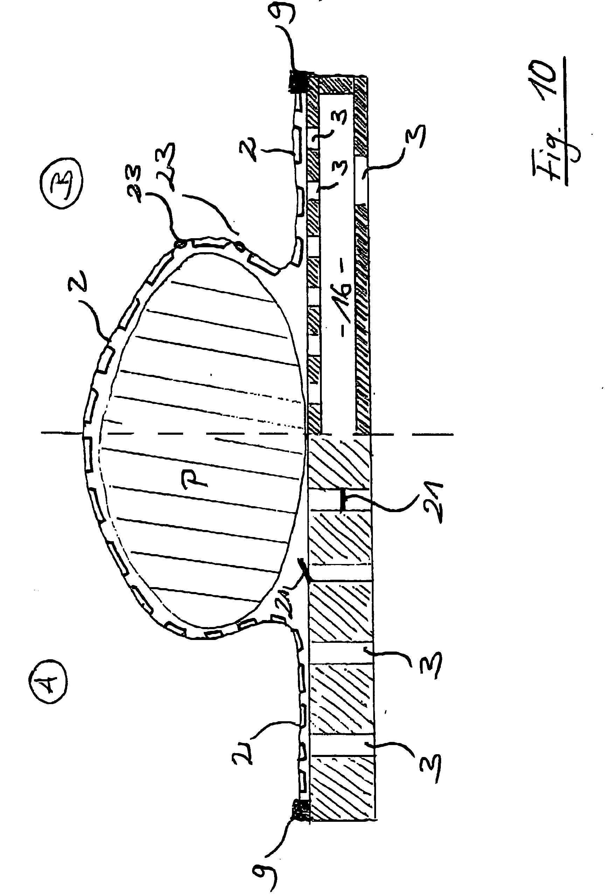

Ein bevorzugtes Ausführungsbeispiel weist eine aus leichtem und röntgendurchlässigem Material gefertigte Körperauflagefläche 1 auf, an die in Modulbauweise (Erweiterbarkeit, Reduzierbarkeit) weitere Plattenteile angeschlossen oder abgenommen werden können. Als derartige Platten können beispielsweise extrudierte Doppelsteg-Kunststoffplatten aus PMMA verwendet werden, wie sie z.B aus Dacheindeckungen bekannt sind. Diese Hohlkammerplatten bilden zugleich in ihrem Innern eine in Fig. 10 rechts dargestellte Absaugkammer 16 aus, die mit einer Vielzahl von Absaugöffnungen 3 in Verbindung steht. Die Körperauflagefläche 1 ist somit leicht und transportabel. Sie kann an ihren Seiten auch mit stabilen Aufhängevorrichtungen 4, beispielsweise Verschraubungen, mit einem Operationstisch verbunden sein. Die Körperauflagefläche 1 kann an diesen Anschlüssen gegenüber dem OP-Tisch in horizontaler und in vertikaler Richtung verstellt und gedreht werden, wobei in jeder Lage ein hohes Maß an Festigkeit gewährleistet ist.

Auf der als Bezugsbasis dienenden Körperauflagefläche 1 ist ein folienartiges Außenelement 2 aufgelegt. Zur Befestigung des Außenelements 2 am umlaufenden Randbereich dienen insbesondere luftdichte Reißverschlüsse, Klett-, Klemm- und Steckverbindungen 9, welche sich einfach und schnell bedienen lassen und einen festen und dichten Abschluß zur Außenluft ermöglichen. Es sei darauf hingewiesen, daß bei geeigneter Materialwahl, insbesondere einer dünnen Kunststoff- oder Metallfolie als Außenelement 2 auch auf eine solche Befestigung am Randbereich verzichtet werden kann, da sich ein solches Außenelement 2 selbst an dem Randbereich der Körperauflagefläche 1 glattflächig anschmiegt und damit abdichtet, insbesondere bei einer Innengummierung oder ähnlichen Beschichtung. Das Außenelement 2 kann jedoch als Kunststoffteil ähnlich einem Deckel oder einer konvexen Schale ebenso wie die (konkave) Körperauflagefläche 1 relativ formstabil ausgebildet sein, die sich dann erst unter Vakuumanlegung an den Körper des Patienten (mit P gekennzeichnet) anlagen.

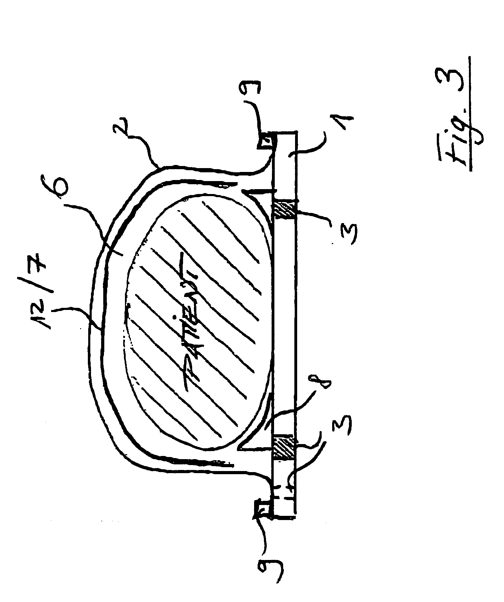

Das Außenelement 2 wird zunächst in Art einer Bettdecke oder eines Tuches locker auf den Patienten P gelegt und am Außenrandbereich der Körperauflagefläche angedrückt bzw. befestigt. Daran anschließend wird über Absaugöffnungen 3 das Medium (insbesondere Luft) in der so gebildeten Unterdruckkammer 6 (vgl. Fig. 3) abgesaugt. Durch die Absaugung legt sich die als Außenelement 2 bevorzugterweise verwendete Folie auf dem Patienten P zunehmend an. Über Meßsonden kann dabei der Unterdruck in der Unterdruckkammer 6 permanent kontrolliert werden.

In das Außenelement 2 eingesetzte Durchgriffsöffnungen 5 mit Manschetten ermöglichen es, daß Körperpartien die Unterdruckkammer 6 verlassen können und damit einer Fixation/Abformung/Komprimierung entzogen sind. Somit können auch die Atemwege und Ohren (Trommelfellinflation) vom angelegten Vakuum freigehalten sein.

Die Unterdruckkammer 6 kann in einfachster Ausführung ein gegenüber der Umgebung abgeschlossenes System bilden. Einmaliges Absaugen und Verschließen des Systems, beispielsweise mittels der in Fig. 10 dargestellten Absperrventile 21 läßt den in der Unterdruckkammer 6 entstandenen Unterdruck lange konstant aufrechterhalten. Eine weitere Möglichkeit, insbesondere bei längeren Operationen ist jedoch, durch die Einbringung von Poren bzw. Luftkanälen 23 das System "halboffen" zu gestalten. Der erwünschte Unterdruck in der Unterdruckkammer 6 kann dann mit einer permanenten Absaugung (unter "Flow") aufrechterhalten werden. Ebenso ist damit die Einbringung von geeigneten Medien (Kühlflüssigkeit, Medikamente, Nährlösungen, usw.) damit möglich. Auch können vom Körper abgegebene Stoffe (Blut, Wundsekrete, ...) damit in vorteilhafter Weise abgesaugt werden. Eine weitere Anwendung wäre die Absaugung von Fettpölsterchen unter der Haut mit postoperativem Aufrechterhalten des Vakuums als Druckverband.

Wie insbesondere in Fig. 3 dargestellt, liegt der Patient P dabei bevorzugt auf einem Auflageelement 8, welches sich zwischen Patient P und Körperauflagefläche 1 befindet. Diese luftdurchlässige Patientenauflage 8 ist relativ weich ausgebildet (z.B. Stoff, Fell, Polster, ...) und dient damit zur Erhöhung des Liegekomforts, kann aber auch eine feste, strukturgebende Form aufweisen (z.B. eine Rinne, konkave Kissen usw.) und dadurch eine reproduzierbare Lagerung bei mehrmaligen Eingriffen möglich machen. Ähnliche Einlageelemente 7, analog den Auflageelementen 8 können auch zwischen Außenelement 2 und dem Patient P angebracht sein. Die Einlage- und Auflageelemente 7 und 8 können frei verschiebbar und damit dem Körper des Patienten P anpaßbar sein, oder auf der Körperauflagefläche 1 befestigt sein. Sie erhöhen die Repostioniergenauigkeit und vergrößern die Anlagefläche auf der Körperauflagefläche 1.

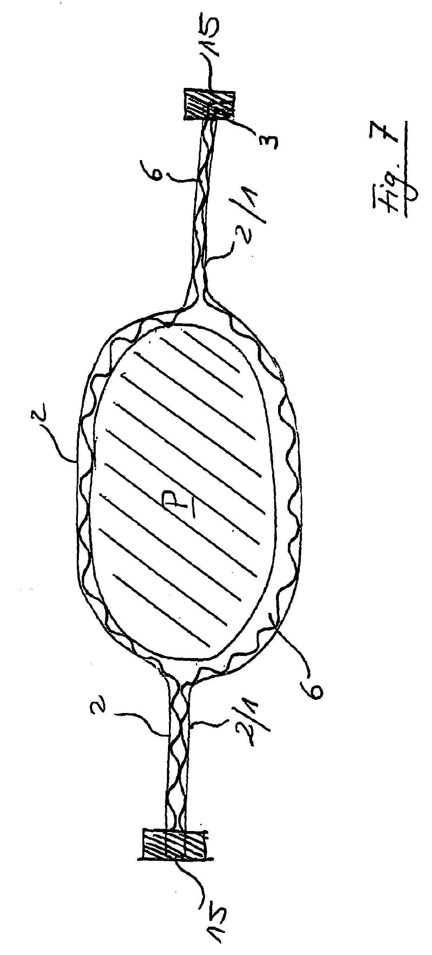

Eine Alternative zur ebenen Körperauflagefläche 1 bietet die Verwendung eines in Fig. 7 dargestellten Rahmens 15, in welchem zwei Außenelemente 2 verankert sind, und damit der Körper (oder einzelne Körperteile) komplett durch die Außenhaut umhüllt ist, der Patient also im fixierten Zustand frei im Rahmen 15 hängt. Mit dieser Technik ist damit der Patient im fixierten Zustand komplett von den Außenelementen 2 umhüllt und es herrscht im fixierten Zustand mit einer zugfesten Folie praktisch gleicher Druck an jeder Körperstelle. Wird andererseits eine starre Körperauflagefläche 1 oder ein nur mäßig dehnbares Außenelement 2 verwendet, kann der Druck auf einigen Körperregionen erhöht werden.

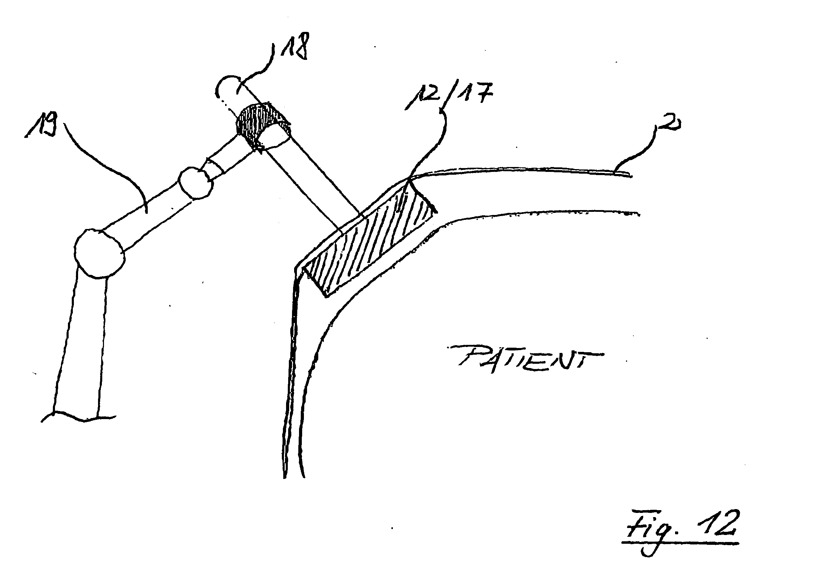

Die Körperauflagefläche 1 und das Außenelement 2 können auch durch Unterkammerung unterteilt werden. Damit können in den einzelnen Kammern unabhängige Druckverhältnisse geschafft werden. Zudem können Zusatzeinrichtungen wie frei positionierbare, stativförmige Abstützelemente oder Haltevorrichtungen 18/19 (vgl. auch Fig. 12) mechanisch, magnetisch oder pneumatisch auf der Körperauflagefläche 1 angebracht werden.

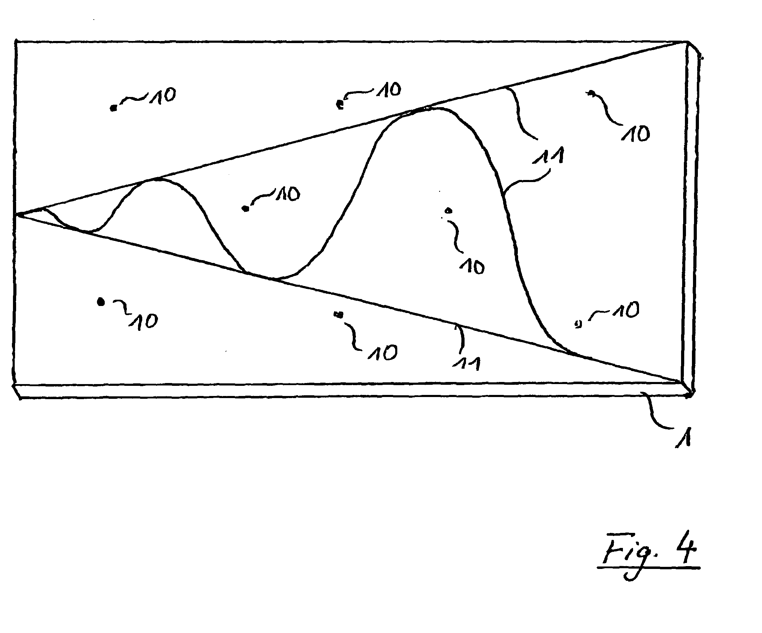

Bei stereotaktischen Operationen sind zudem Bezugspunkte wesentlich, wobei die vorstehend beschriebenen Elemente auch als Halterung für Eichpunkte 10 dienen können. Die in Fig. 4 dargestellten Eichpunkte in Form von Bleikügelchen können als einfaches Referenzsystem dienen. Daneben können jedoch auch andere Sensorentypen verwendet werden z. B. optische Reflektoren, radioaktive oder induktive Sender. Denkbar sind auch quer über die Körperauflagefläche 1 oder das Außenelement 2 laufende Eichlinien 11, die durch ihre Geometrie genaue Rückschlüsse auf die Patientenlage zulassen. Solche Eichsysteme oder Markersysteme können somit auf der Körperauflagefläche 1, dem Außenelement 2, aber auch auf den Haltearmen 19 oder Schienungen 12 bzw. Schalungen 17 (vgl. Fig. 12) angebracht sein. Die genannten Schienungen 12 (z.B. Streifen oder Bänder) können insbesondere bei Operationen in schlecht fixierbaren Regionen die Fixation der betroffenen Körperregion erhöhen (z.B. bei Weichteilen).

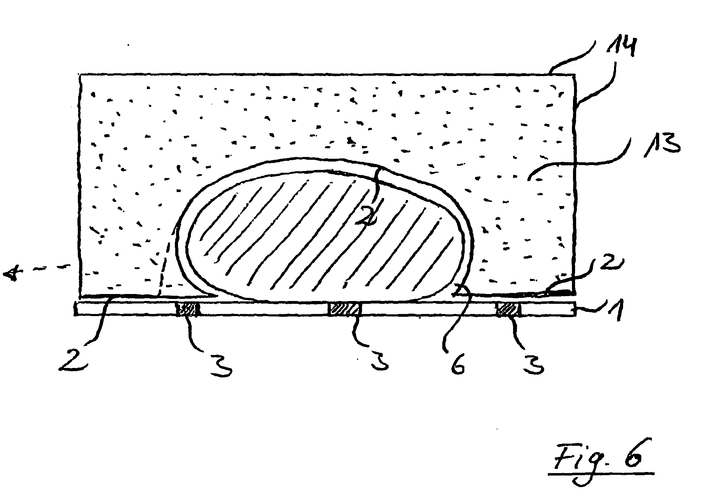

Wie in Fig. 6 dargestellt, können über einen über dem Außenelement 2 angebrachten Abformbehälter 14 (geschlossen oder offen) Abformmaterialien 13 eingefüllt werden, insbesondere PU-Schaum, Gips oder Styroporkügelchen, welche dann für einen Körperabdruck verfestigt werden können. In das Abformmaterial 13 können ebenfalls Einlageelemente (Streifen, Gummimatten) eingelegt werden. Nach Aushärten des Abformmaterials 13 können einzelne Einlageelemente abgezogen werden und bei einer späteren Repositionierung als Unterdruckkanal innerhalb des so gebildeten patientenindividuell hergestellten Außenelements 2 dienen.

Wie in Fig. 10 dargestellt, können die Absaugöffnungen 3 entweder direkt in die Vakuumkammer 6 führen oder über eine Absaugkammer 16 an die Vakuumkammer 6 angeschlossen sein. Bei letztgenannter Ausführung können mehrere mit der Absaugkammer 16 verbundene Absaugöffnungen 3 mit exakt gleichem Unterdruck homogen betrieben werden. Eine solche Absaugkammer 16 bzw. die beschriebenen Absaugöffnungen 3 (insbesondere feine Perforationen in der Oberwand der Körperauflagefläche 1) befinden sich nicht notwendigerweise nur in der Körperauflagefläche 1, sondern können auch in das Außenelement 2 eingebracht oder integriert sein.

Im folgenden soll die praktische Anwendung der vorgeschlagenen Vorrichtung beschrieben werden:
a) zur (reinen) Fixation:
   - zur Ruhigstellung bei diagnostischen Verfahren (CT, MRI);
   - bei Operationen, Biopsieentnahmen, Bestrahlungen, usw.;
   - zur Ruhigstellung von Patienten: z.B. bei psychiatrisch erkrankten Patienten ("Vakuum-Zwangsjacke") oder zur Vermeidung von Verletzungen bei somnolenten/komatösen Patienten;
   - zur Ruhigstellung von Körperteilen bei (orthopädischen und chirurgischen) Verletzungen mit dem Vorteil der simultanen Absaugung von Wundsekret und/oder Komprimierung von Blutungen;
b) zur Komprimierung:
   - Schockbehandlung bei diversen Schockarten;
   - Aufbewahrung von Amputaten;
   - Unterdrückung von Blutungen bei Gefäßverletzungen/Amputationen;
   - zur Simulation künstlicher Druckverhältnisse (z.B. beim Astronauten- oder Tauchertraining);
   - postoperative Nachbehandlung (Kompressionsverband z.B. auch bei Tätowierungen, Anlegen von Kältemitteln);
c) zur Abformung von Körperteilen:
   - exakte Anbringung von Abformelementen und -massen (z.B. als PU-Schaum);
d) zur reproduzierbaren Fixation:
   - bei wiederholten Eingriffen, bei welchen eine immer gleiche Patientenlagerung erforderlich ist ( insbesondere sämtliche stereotaktischen Anwendungen: z.B. Neurochirurgie, Brachytherapie, fraktionierte Bestrahlung);
Die Vorteile lassen sich wie folgt zusammenfassen:
a) für die Fixation:
   - homogene Kompression des Körpers (falls erwünscht);
   - maximale Sicht- und Bewegungsfreiheit für den Operateur;
   - Positionierung des Körpers in variablen Lagen bei Eingriffen;
   - keine Hautverschiebung und Lageverschiebungen möglich;
   - Anbringungsmöglichkeit von Instrumenten (z.B. Endoskop);
b) für reproduzierbare Fixation:
   - Definition von exakten Eichpunkten für sterectaktische Eingriffe (optische, radioaktive oder induktive Sensoren oder Sender, etc.) in immer gleicher Relation zum Körper;
   - geringer Materialaufwand;
c) für die Abformung:
   - kaum Faltenbildung des Folien-Außenelements;
   - höchste Genauigkeit durch exaktes Anlegen mittels Vakuum;
d) allgemeine Vorteile:
   - höchste Stabilität bei geringem Gewicht;
   - Tauglichkeit für diagnostische (CT, MRI) Verfahren;
   - gleichmäßiger Druck an allen Stellen oder erhöhter Druck an Stellen mit eingebrachten Schienelementen;
   - stufenlose Druckregulierung möglich;
   - platzsparender Aufbau;
   - einfache und zeitsparende Handhabung durch die Unterdruckanlegung und daher besonders patientenschonend und hygienisch;
   - hohe Patientensicherheit durch Kontrolle des Unterdruckes in den Unterdruckkammern und der Druckbelastung des Patienten; zudem sofortige Befreiungsmöglichkeit und non-invasive Fixierung.

## Patentansprüche

1. Vorrichtung zur Fixierung und/oder Komprimierung und/oder Abformung von Körper-(teilen), insbesondere des menschlichen Körpers, **gekennzeichnet durch**:
- eine Körperauflagefläche (1),
- mindestens ein Außenelement (2), welches frei auf der Körperauflagefläche (1) positionierbar ist und mit der Körperauflagefläche (1) eine den Körper umschließende Unterdruckkammer (6) bildet, und
- mindestens eine Absaugöffnung (3), welche mit der Unterdruckkammer (6) in Verbindung steht.

2. Vorrichtung nach Anspruch 1,
**dadurch gekennzeichnet, daß**
die Körperauflagefläche (1) luftdicht mit dem Außenelement (2) verbindbar ist.

3. Vorrichtung nach Anspruch 1 oder 2,
**dadurch gekennzeichnet, daß**
die Körperauflagefläche (1) weitgehend starr ausgebildet ist und in ihrer Oberfläche eine Vielzahl von Absaugöffnungen (3) aufweist.

4. Vorrichtung nach einem der Ansprüche 1 bis 3,
**dadurch gekennzeichnet, daß**
das Außenelement (2) flexibel, insbesondere folienartig ausgebildet ist.

5. Vorrichtung nach einem der Ansprüche 1 bis 4,
**dadurch gekennzeichnet, daß**
die Körperauflagefläche (1) in Auflagerichtung elastisch ausgebildet ist.

6. Vorrichtung nach einem der Ansprüche 1 bis 5,
**dadurch gekennzeichnet, daß**
das Außenelement (2) in Anlagerichtung elastisch und in Querrichtung zugfest ausgebildet ist.

7. Vorrichtung nach einem der Ansprüche 1 bis 6,
**dadurch gekennzeichnet, daß**
die Körperauflagefläche (1) an einem Eingriffs- oder Operationstisch anbringbar ist, insbesondere mittels einer Schwenkaufhängung (4).

8. Vorrichtung nach einem der Ansprüche 1 bis 7,
**dadurch gekennzeichnet, daß**
das Außenelement (2) am Randbereich der Körperauflagefläche (1) ansaugbar ausgebildet ist.

9. Vorrichtung nach einem der Ansprüche 1 bis 8,
**dadurch gekennzeichnet, daß**
Zusatzeinrichtungen (22) mechanisch, pneumatisch oder magnetisch repositionierbar an der Körperauflagefläche (1) anbringbar sind.

10. Vorrichtung nach einem der Ansprüche 1 bis 9,
**dadurch gekennzeichnet, daß**
das Außenelement (2) am Randbereich der Körperauflagefläche (1) zusätzlich mechanisch, pneumatisch und/oder magnetisch befestigt ist.

11. , Vorrichtung nach einem der Ansprüche 1 bis 10,
**dadurch gekennzeichnet, daß**
die Absaugöffnungen (3) als einzelne Absaugkanäle ausgebildet sind oder an mindestens eine gemeinsame Absaugkammer (16) angeschlossen sind.

12. Vorrichtung nach einem der Ansprüche 1 bis 11,
**dadurch gekennzeichnet, daß**
die Absaugkammer(-n) (16) in der Körperauflagefläche (1) und/oder dem Außenelement (2) untergebracht ist/sind.

13. Vorrichtung nach einem der Ansprüche 1 bis 12,
**dadurch gekennzeichnet, daß**
die Körperauflagefläche (1) in einzelne Absaugkammern (16) unterteilt ist.

14. Vorrichtung nach einem der Ansprüche 1 bis 13,
**dadurch gekennzeichnet, daß**
das Außenelement (2) in einzelne Unterdruckkammern (6) unterteilt ist.

15. Vorrichtung nach einem der Ansprüche 1 bis 14,
**dadurch gekennzeichnet, daß**
das Außenelement (2), insbesondere als Metallfolie vollkommen luftdicht abschließt oder mittels Poren oder Zustromkanälen (23) teilweise luftdurchlässig sind.

16. Vorrichtung nach einem der Ansprüche 1 bis 15,
**dadurch gekennzeichnet, daß**
die Körperauflagefläche (1) und/oder das Außenelement (2) aus einzelnen Modulen bestehen und aneinanderfügbar sind.

17. Vorrichtung nach einem der Ansprüche 1 bis 16,
**dadurch gekennzeichnet, daß**
das Außenelement (2) in Material und Aufbau der Körperauflagefläche (1) ähnlich oder gleich ist.

18. Vorrichtung nach einem der Ansprüche 1 bis 17,
**dadurch gekennzeichnet, daß**
die Absaugöffnungen (3) und/oder Zuluftkanäle (23) und/oder Absaugkammern (16) mittels Absperrventilen (21) verschließbar sind.

19. Vorrichtung nach einem der Ansprüche 1 bis 18,
**dadurch gekennzeichnet, daß**
Einlageelemente (7) zwischen dem Körper (P) und dem Außenelement (2) und/oder Körperauflagefläche (1) einlegbar sind.

20. Vorrichtung nach Anspruch 19,
**dadurch gekennzeichnet, daß**
die Einlageelemente (7) als Schienung (12) oder Schalung (17) in das Außenelement (2) oder die Körperauflagefläche (1) eingearbeitet sind.

21. Vorrichtung nach einem der Ansprüche 1 bis 20,
**dadurch gekennzeichnet, daß**
vorgeformte oder individuell geformte Patientenauflagen (8) an der Körperauflagefläche (1) und/oder dem Außenelement (2) anlegbar sind.

22. Vorrichtung nach Anspruch 21,
**dadurch gekennzeichnet, daß**
als Patientenauflage (8) Elemente zur Verringerung des Reibungswiderstands, insbesondere Gleitfolien, oder zur Erhöhung des Reibungswiderstands, insbesondere eine Gummimatte, an dem Außenelement (2) und/oder der Körperauflagefläche (1) anbringbar sind.

23. Vorrichtung nach einem der Ansprüche 1 bis 22,
**dadurch gekennzeichnet, daß**
ein Abformbehälter (14) zur Aufnahme von Abformmaterial (13) auf dem Außenelement (2) anbringbar ist.

24. Vorrichtung nach einem der Ansprüche 1 bis 23,
**dadurch gekennzeichnet, daß**
am Randbereich der Körperauflagefläche (1) Befestigungen (9) für das Außenelement (2), insbesondere zur Fixierung auf einem Operationstisch angebracht sind.

25. Vorrichtung nach einem der Ansprüche 1 bis 24,
**dadurch gekennzeichnet, daß**
die Elemente (1, 2,....) der Vorrichtung aus röntgendurchlässigem und/oder nicht-ferromagnetischem Material bestehen.

26. Vorrichtung nach einem der Ansprüche 1 bis 25,
**dadurch gekennzeichnet, daß**
auf der Körperauflagefläche (1) Markierungen und/oder Steckbohrungen zur Repositionierung eines Patienten (P) angebracht sind.

27. Vorrichtung nach einem der Ansprüche 1 bis 26,
**dadurch gekennzeichnet, daß**
Überstreck-/Dahn-/Stauchvorrichtungen für den Körper oder für Körperteile an der Körperauflagefläche (1) vorgesehen sind.

28. Vorrichtung nach einem der Ansprüche 1 bis 27,
**dadurch gekennzeichnet, daß**
Halteelemente (19), insbesondere als magnetische oder als pneumatische Halterungen auf der Körperauflagefläche (1) anbringbar sind und als Trag- und Aufnahmevorrichtungen für medizinische Geräte, insbesondere endoskopische Apparaturen, Wundhaken, Handauflageflächen oder stereotaktische Navigationshilfen vorgesehen sind.

29. Vorrichtung nach einem der Ansprüche 1 bis 28,
**dadurch gekennzeichnet, daß**
Fixationsvorrichtungen (22) für den Körper oder für Körperteile an die Körperauflagefläche (1) anschließbar sind.

30. Vorrichtung nach einem der Ansprüche 1 bis 29,
**dadurch gekennzeichnet, daß**
Druckmesseinrichtungen zur Kontrolle der (Unter-) Druckverteilung in der Unterdruckkammer (6) vorgesehen sind.

31. Vorrichtung nach einem der Ansprüche 1 bis 30,
**dadurch gekennzeichnet, daß**
ein Patientenbefreiungsmechanismus mit plötzlichem Abbau des Unterdrucks in der Unterdruckkammer (6) vorgesehen ist.

32. Vorrichtung nach einem der Ansprüche 1 bis 31,
**dadurch gekennzeichnet, daß**
das Außenelement (2) und/oder die Körperauflagefläche (1) an wenigstens einer Stelle eine Durchgrifföffnung (5) aufweist, die ringsum abgedichtet sind.

33. Vorrichtung nach einem der Ansprüche 1 bis 32,
**dadurch gekennzeichnet, daß**
in dem Außenelement (2) oder der Körperauflagefläche (1) ringsum abgedichtete Operationsöffnungen (20) ausgebildet sind.

34. Vorrichtung nach einem der Ansprüche 1 bis 33,
**dadurch gekennzeichnet, daß**
wenigstens ein einstellbares Eichsystem (10/11), das zur Positionsfestlegung von Referenzpunkten vorgesehen ist, in die Vorrichtungselemente (1, 2) eingearbeitet ist.

35. Vorrichtung nach einem der Ansprüche 1 bis 34,
**dadurch gekennzeichnet, daß**
das Außenelement (2) und die Körperauflagefläche (1) eine Einheit bilden, welche beutel- oder schlauchartig den Körper oder einzelne Körperteile umschließt.

36. Vorrichtung nach einem der Ansprüche 1 bis 35,
**dadurch gekennzeichnet, daß**
zur hängenden/schwebenden Fixation des Körpers von beiden Seiten die Körperauflagefläche (1) und das Außenelement (2) in eine Rahmenaufhängung (15) eingespannt sind.

37. Vorrichtung nach einem der Ansprüche 1 bis 36,
**dadurch gekennzeichnet, daß**
die Körperauflagefläche (1)/Außenelement (2) aus einem anfänglich flexiblen und nach Aushärtung irreversiblen Material besteht, insbesondere einem Thermo-Abformmaterial.

38. Verfahren zur Fixation von Körper-(teilen) insbesondere des menschlichen Körpers, mittels wenigstens einer Körperauflagefläche (1) und wenigstens einem Außenelement (2), welches mittels Unterdruck an der Körperauflagefläche (1) angesaugt wird, wodurch der eingeschlossene Körper fixiert wird.

39. Verfahren zur Kompression von Körper-(teilen) insbesondere des menschlichen Körpers, mittels wenigstens einer Körperauflagefläche (1) und wenigstens einem Außenelement (2), welches mittels Unterdruck an der Körperauflagefläche (1) angesaugt wird, wodurch der eingeschlossene Körper mit definiertem Druck komprimiert wird.

40. Verfahren zur Abformung von Körper-(teilen) insbesondere des menschlichen Körpers, mittels wenigstens einer Körperauflagefläche (1) und wenigstens einem Außenelement (2), welches mittels Unterdruck an der Körperauflagefläche (1) angesaugt wird, und dann der eingeschlossene Körper abgeformt wird.

41. Verfahren zur Festlegung von Eichpunkten in Relation zum Körper-(teilen), insbesondere des menschlichen Körpers, mittels wenigstens einer Körperauflagefläche (1) und wenigstens einem Außenelement (2), welches mittels Unterdruck an der Körperauflagefläche (1) angesaugt wird, wodurch der eingeschlossene Körper relativ zu Eichpunkten (10) positioniert ist.
